# EUROPÄISCHE PATENTSCHRIFT

(11) **EP 3 427 694 B1**
(45) Veröffentlichungstag und Bekanntmachung des Hinweises auf die Patenterteilung: **04.12.2019**
(21) Anmeldenummer: 18174666.0
(22) Anmeldetag: 29.05.2018
(51) Int. Cl.: A61F 2/18

(54) **SELBSTKLEMMENDE KOPFPLATTE FÜR EINE GEHÖRKNÖCHELCHENPROTHESE**
SELF-LOCKING HEAD PLATE FOR AN AUDITORY OSSICLE PROSTHESIS
PLAQUE SUPÉRIEURE À AUTOSERRAGE POUR UNE PROTHÈSE D'OSSELET

(30) Priorität: 13.07.2017 DE 102017115806
(43) Veröffentlichungstag der Anmeldung: 16.01.2019
(73) Patentinhaber: Heinz Kurz GmbH, 72144 Dusslingen (DE)
(72) Erfinder: Gäckle, Markus, 75378 Bad Liebenzell (DE); Lang, Axel, 72770 Reutlingen (DE)
(74) Vertreter: Kohler Schmid Möbus Patentanwälte

(56) Entgegenhaltungen:
- EP-A2- 1 054 573
- EP-B1- 0 998 884
- WO-A1-2018/052866
- DE-U1-202008 003 887
- DE-U1-202008 003 890

## Beschreibung

Die Erfindung betrifft die Kopfplatte einer Gehörknöchelchenprothese, welche mindestens ein Glied oder Teile eines Gliedes der Gehörknöchelchenkette ersetzt oder überbrückt, wobei die Kopfplatte zur mechanischen Anlage am Trommelfell und/oder am Hammergriff ausgebildet ist und an einem Ende der Gehörknöchelchenprothese als ein erstes Befestigungselement montierbar ist, während am anderen Ende der Prothese ein zweites Befestigungselement zur mechanischen Verbindung mit einem Glied oder Teilen eines Gliedes der Gehörknöchelchenkette oder direkt mit dem Innenohr sowie ein entlang einer Längsachse die beiden Befestigungselemente Schall leitend miteinander verbindendes Verbindungselement mit zumindest einem Schaftförmigen Ende vorgesehen ist, wobei die Kopfplatte einen radial äußeren, die Längsachse umgebenden Ringbereich aufweist, an dessen Innenrand ein einzelner, längs einer gekrümmten Kurve verlaufender Steg an zwei voneinander räumlich getrennten Angriffspunkten fest mit dem Ringbereich verbunden ist, wobei seitlich neben dem Steg zwei durch den Steg getrennte Durchbrüche innerhalb des Ringbereichs offen bleiben, wobei in einem mittleren Abschnitt des Stegs eine Durchgangsbohrung zur Aufnahme des Schaftförmigen Endes des Verbindungselements sowie ein von einem der Durchbrüche zur Durchgangsbohrung verlaufender Schlitz zum seitlichen Einführen des Schaftförmigen Endes des Verbindungselements vom Durchbruch aus in die Durchgangsbohrung vorgesehen ist, wobei die Durchgangsbohrung nach dem Einführen des Schaftförmigen Verbindungselements verengbar ist, um dieses in der Durchgangsbohrung zu fixieren.

Eine derartige Vorrichtung ist bekannt aus EP 0 998 884 B1, DE 20 2008 003 887 U1, DE 20 2008 003 890 U1 und WO 2018/052866.

### Hintergrund der Erfindung

Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell beziehungsweise am Hammergriff anliegt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten GehörknöchelchenProthesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Bei der Implantation von Gehörknöchelprothesen muss vor der Implantation die Länge der Prothese individuell auf den jeweiligen Patienten abgestimmt werden. Da man die erforderliche Länge aber erst während der Operation bestimmen kann, musste zunächst für jede Operation entweder ein Satz von Prothesen unterschiedlicher Länge oder eine Prothese mit variabler Schaftlänge bereitgestellt werden.

Bei weiteren bekannten, längenveränderlichen Gehörknöchelprothesen ist der Schaft der Prothese in mehrere aufeinander folgende Abschnitte unterteilt, wobei die aufeinander folgenden Abschnitte mittels einer Querschnittsverjüngung als Sollbruchstellen voneinander getrennt sind. Weil bei den filigranen Gehörknöchelprothesen jedoch im Bereich von Zehntel und Hundertstel Millimetern gearbeitet wird, ist die Fertigung der Sollbruchstellen verhältnismäßig aufwendig und damit teuer.

Demgegenüber verbessert die Gehörknöchelchenprothese nach der eingangs zitierten EP 0 998 884 B1 diesen Stand der Technik dahingehend, dass das Ablängen des Schaftes auf die individuelle Länge mit einem geringen Fertigungsaufwand für den Schaft und damit kostengünstig erfolgen kann. Dies wird dadurch erreicht, dass die Kopfplatte eine Durchgangsbohrung zur Aufnahme des Schaftes aufweist, durch die der längliche Schaft hindurchgeschoben werden kann, sodass er auf der Außenseite der Kopfplatte übersteht und dort abgelängt werden kann, wobei die Bohrung verengbar ist, um den Schaft zu fixieren. Anschließend nach der Verkürzung des Schaftes wird die Bohrung der Kopfplatte verengt und dadurch der Schaft sicher fixiert. Danach kann die angepasste Prothese dem Patienten implantiert werden. Die aus der EP 0 998 884 B1 bekannte Gehörknöchelprothese ist somit rasch und stufenlos an die anatomischen Gegebenheiten des Patienten anpassbar.

Zur Verengung der Bohrung in der Kopfplatte gibt es gemäß EP 0 998 884 B1 verschiedene Möglichkeiten: So kann der Umfang der Bohrung durch einen Schlitz unterbrochen und der Schlitz und damit die Bohrung über einen verformbaren Steg durch Verstemmen des Steges verengbar sein. Dadurch kann der Schaft mit wenig Aufwand rasch an der Kopfplatte fixiert werden. Aus demselben Grund kann die Bohrung in der Mitte eines Schlitzes liegen und die Bohrung durch Zusammendrücken des Schlitzes verengbar sein. Die Bohrung kann auch in der Mitte von zwei sich kreuzenden Schlitzen liegen, wobei die Bohrung einen kleineren Durchmesser als der Schaftdurchmesser aufweist.

### Nachteile des Standes der Technik

Ein wesentlicher Nachteil dieser bekannten Kopfplatte liegt jedoch darin, dass bei deren Befestigung am Schaft eine Klemmkraft mittels aktiver plastischer Deformation im Bereich der Durchgangsbohrung erzeugt werden muss. Diese Kraft fällt in der Praxis -je nach Anwender und dessen aktueller Konstitution- stets völlig unterschiedlich aus und ist in keinem Fall gleich. Daher ist es auch nicht möglich, bei verschiedenen Operationen desselben Mittelohrchirurgen und erst recht bei unterschiedlichen Ärzten ein -auch nur halbwegs- vergleichbares oder gar reproduzierbares mechanisches Verhalten beim Einfügen des Schaftes in die Kopfplatte zu erreichen. Daher ist die Befestigung des Schaftes in der Kopfplatte gemäß der EP 0 998 884 B1 prinzipiell nicht validierbar und noch viel weniger automatisierbar.

Zudem ist eine sichere Klemmung mit anschließendem andauernden Halt des Schaftes in der Kopfplatte wegen der erforderlichen plastischen Verformung der Kopfplatte nur ein einziges Mal erzeugbar. Falls danach noch eine Korrektur vorgenommen werden soll/muss, kann dies ausschließlich mit einer neuen Kopfplatte, in der Regel sogar nur mit einem neuen Schaft, geschehen. Die vorher verwendete Kopfplatte hat dann einen "Totalschaden" erlitten und kann nur noch weggeworfen werden.

### Aufgabe der Erfindung

Aufgabe der vorliegenden Erfindung ist es demgegenüber, eine gattungsgemäße Vorrichtung der eingangs beschriebenen Art mit möglichst einfachen technischen Mitteln dahingehend zu verbessern, dass unaufwändig und kostengünstig ein objektiv reproduzierbares mechanisches Klemmverhalten beim Befestigen des Schaftes in der Kopfplatte mit insbesondere gleichbleibender, vorgebbarer Klemmkraft erreicht sowie -etwa bei einer suboptimalen Positionierung- eine mehrfache Wiederholbarkeit des Klemmvorgangs mit derselben Kopfplatte ermöglicht wird.

### Kurze Beschreibung der Erfindung

Erfindungsgemäß wird diese Aufgabe auf ebenso überraschend einfache wie wirkungsvolle Art und Weise dadurch gelöst, dass der Steg als monolithisches Hebelsystem ausgebildet ist und einen ersten langen Hebelabschnitt aufweist, der vom ersten Angriffspunkt des Steges am Ringbereich zu einer Seite eines im Steg angeordneten gemeinsamen Biegegelenks verläuft, von dessen anderer Seite ein zweiter langer Hebelabschnitt zum zweiten Angriffspunkt des Steges am Ringbereich verläuft, dass im Steg ein erster kurzer Hebelabschnitt, der eine geringere Länge aufweist als der erste lange Hebelabschnitt, sowie ein zweiter kurzer Hebelabschnitt, der eine geringere Länge aufweist als der zweite lange Hebelabschnitt, vorgesehen sind, wobei der erste kurze Hebelabschnitt vom Verbindungspunkt des ersten langen Hebelabschnitts mit dem gemeinsamen Biegegelenk zu einer ersten Seite des Schlitzes und der zweite kurze Hebelabschnitt vom Verbindungspunkt des zweiten langen Hebelabschnitts mit dem gemeinsamen Biegegelenk zu einer zweiten Seite des Schlitzes verlaufen, und dass der erste Angriffspunkt des Steges am Ringbereich, das gemeinsame Biegegelenk und der zweite Angriffspunkt des Steges am Ringbereich nicht-kollinear angeordnet sind, so dass durch Betätigung des ersten und zweiten langen Hebelabschnitts über das gemeinsame Biegegelenk der erste und zweite kurze Hebelabschnitt betätigt werden.

Damit gelingt eine Übertragung der elastischen Deformationskraft vom Ringbereich auf die Durchgangsbohrung (Klemmbereich) über das Hebelsystem, das als Kraftverstärkersystem wirkt. Die Klemmung kann wieder geöffnet werden ohne den Schaft oder die Prothese zu beschädigen. Weiter wird mit der Erfindung das System validierbar und somit die entscheidenden Bearbeitungsschritte beim Implantieren der Prothese objektiv reproduzierbar und -zumindest teilweise- auch automatisierbar. Außerdem können damit auf relativ simple Weise die Vorteile der oben beschriebenen gattungsgemäßen Gehörknöchelchenprothese, wie sie in EP 0 998 884 B1 beschrieben ist, im Wesentlichen beibehalten werden.

### Genaue Wirkungsweise der Erfindung

Eine gemäß der vorliegenden Erfindung modifizierte Mittelohrprothese (Total- und Partialprothese) umfasst als zentrales Bauteil einen Schaft, an dessen einem Ende eine Verbindungsmimik (Glocke, Clip, usw.) am anderen Ende ein Verbindungsstück in Form einer erfindungsgemäßen Kopfplatte als Verbindung zum Trommelfell bzw. zum Malleus (Hammer) oder zu beidem.

Die erfindungsgemäße Kopfplatte ist -wie auch schon beim Stand der Technik- auf dem Schaft durch Klemmung fixiert. Die Klemmkraft wird aber nunmehr erzeugt durch elastische materialeigene Federkraft umgesetzt durch eine "monolithische" Hebelmechanik.

Durch Einleitung einer Kraft wird eine bestimmte elastische Verformung der Kopfplatte bewirkt und dadurch die Hebelmechanik bewegt und die Klemmung am Schaft gelöst. So kann die Position der Kopfplatte auf dem Schaft -innerhalb von durch die Geometrie vorgegebenen Grenzen- verschoben werden. Wird dann die eingeleitete Verformungskraft wieder weggenommen, nimmt die Kopfplatte aufgrund ihrer Elastizität wieder ihre Ausgangsform an. Die auf diese Weise auf die Hebelmechanik übertragenen Kräfte sorgen für eine sichere Klemmung am Schaft. Die Hebelmechanik wirkt unter anderem als Kraftverstärkung. Sie verstärkt die "Rückstellkraft" der Kopfplatte und überträgt diese auf den Schaft, wodurch eine sehr große Flächenpressung und somit eine sehr feste Klemmung entsteht.

Die erfindungsgemäße neue Kopfplatte hat gegenüber bekannten, auf dem Markt derzeit erhältlichen Tympanoplastikprothesen entscheidende Vorteile: Bei der neuen selbsthemmenden Kopfplatte -die vorzugsweise aus superelastischem Nitinol hergestellt ist- muss keine Quetschbewegung innerhalb der Kopfplatte mehr durchgeführt werden um die Kopfplatte auf dem Schaft zu fixieren. Durch Druck auf die Außenkontur wird auf die Hebelübersetzung ein großer Weg mit geringer Kraft auf einen sehr kleinen Weg mit großer Kraft übersetzt. So wurden in Abzugsversuchen weitaus größere Haltekräfte erreicht als bei der bisherigen am Markt befindlichen Tympanoplastikprothesen.

### Bevorzugte Ausführungsformen der Erfindung

Eine besonders bevorzugte Ausführungsform der erfindungsgemäßen Gehörknöchelchenprothese zeichnet sich dadurch aus, dass der erste und der zweite lange Hebelabschnitt jeweils einstückig am ersten bzw. zweiten Angriffspunkt des Steges mit dem Ringbereich verbunden sind. Die Einstückigkeit reduziert den Montageaufwand sowie die Herstellungskosten und erhöht die Präzision.

Bei weiteren vorteilhaften Ausführungsformen sind der erste und der zweite Angriffspunkt des Steges am Ringbereich jeweils als weitere Biegeelemente ausgebildet. Dies trägt ebenfalls zu einer erhöhten Präzision bei.

Bei einer bevorzugten Klasse von Ausführungsformen der Erfindung sind der erste und der zweite Angriffspunkt des Steges am Ringbereich jeweils als weitere Biegeelemente ausgebildet. Auch dies erhöht die Präzision des gesamt-Systems und damit dessen Validierbarkeit.

Besonders vorteilhaft sind Weiterbildungen dieser Klasse von Ausführungsformen, bei welchen der erste und der zweite lange Hebelabschnitt jeweils in einem Bereich vor dem ersten bzw. dem zweiten Angriffspunkt eine geringere Stegbreite aufweisen als in ihren übrigen Bereichen, was die Wirksamkeit des Biegegelenks entscheidend erhöhen kann.

Weitere bevorzugte Ausführungsformen der Erfindung zeichnen sich dadurch aus, dass der Steg in einem Bereich zwischen dem ersten Angriffspunkt und dem zweiten Angriffspunkt am Ringbereich, der auch das gemeinsame Biegegelenk umfasst, im Wesentlichen U-förmig gestaltet ist. Diese U-Form ist in der Praxis die ideale Form, um die besten Hebelanordnung im System zu erhalten.

Besonders bevorzugt ist auch eine Ausführungsform der erfindungsgemäßen Kopfplatte, bei der der Schlitz im Steg auch jenseits der Durchgangsbohrung bis zum gemeinsamen Biegegelenk fortgesetzt ist. Dadurch wird die Öffnungskraft reduziert und die Betätigung erleichtert, weil der Einfluss der Steifigkeit des gemeinsamen Biegegelenks reduziert wird. Es ergibt sich damit auch ein geringeres Risiko eines Material-Risses wegen zu starker Deformation.

Weitere vorteilhafte Ausführungsformen der erfindungsgemäßen Kopfplatte zeichnen sich dadurch aus, dass der radial äußere, die Längsachse umgebende Ringbereich kreisrund oder oval gestaltet ist. Durch die ovale oder runde Form des Ringbereichs erhält man im Ringbereich eine ideale Entlastungskraft sowie Hebelkraftverhältnisse, welche im Wesentlichen nur durch die Materiaeigenschaften und die Dimensionierung beeinflusst sind.

Bevorzugt sind auch Ausführungsformen der Erfindung, bei denen die Durchgangsbohrung auf ihrem Umfang gezackt gestaltet ist. Durch einen punktuellen Kontakt im Klemmbereich (Durchgangsbohrung) erhöht sich die Flächenpressung und somit die Haltekraft, die auf den Schaft wirkt.

Bei weiteren vorteilhaften Ausführungsformen der erfindungsgemäßen Kopfplatte weisen der erste bzw. der zweite lange Hebelabschnitt zum ersten bzw. zum zweiten kurzen Hebelabschnitt jeweils ein Hebelverhältnis ≥ 1,5, vorzugsweise zwischen 2 und 6, auf. Damit ergibt sich ein nahezu ideales Kraftverstärkersystem (Hebelgesetz).

In der Regel wird bei der erfindungsgemäßen Kopfplatte die Durchgangsbohrung einen lichten Durchmesser zwischen 0,1mm und 0,4mm, vorzugsweise von etwa 0,2mm, aufweisen. Dies erlaubt auch die Aufnahme eines Schaftes mit geringem Durchmesser und/oder geringer mitschwingender Masse, und zwar bei geringer Kontaktfläche. Somit ergibt sich eine besonders große Flächenpressung und mithin eine gute Haltekraft.

Der erfindungsgemäß vorgesehene Schlitz im Steg der Kopfplatte kann eine Schlitzbreite zwischen 0,03mm und 0,15mm, vorzugsweise von etwa 0,06mm, aufweisen. Dies stellt eine ausreichend große Kontaktfläche zur Klemmung des Schaftes in der Durchgangsbohrung sicher.

Die Plattendicke der Kopfplatte wird in der Regel zwischen 0,1mm und 0,5mm, vorzugsweise etwa 0,25mm betragen, was sich in der Praxis oft bewährt hat.

Um optimale Wirkungen zu erzielen, sollte die erfindungsgemäße Kopfplatte aus einem hochfesten und elastischen Material mit einer minimalen Festigkeit ≥ 450N/mm2 sowie einer minimalen Elastizität ≥ 0,5%, vorzugsweise ≥ 2%, aufgebaut sein. Damit wird unter anderem auch die Klemmung des Schafts aufgrund der elastischen Verformungsenergie des Ringbereichs optimiert, welche bei Entlastung über das monolithische Hebelsystem auf die Klemmung des Schafts mit Kraftverstärkung wirkt.

Bei besonders vorteilhaften Weiterbildungen dieser Klasse von Ausführungsformen der Erfindung ist die Kopfplatte aus einem Metall oder einer Metalllegierung, insbesondere aus Nitinol und/oder aus amorphem Metallglas und/oder aus Metallkeramik und/oder aus einem hochfesten und elastischen Polymer, insbesondere aus faserverstärktem Kunststoff, vorzugsweise aus Karbonfaser-Material aufgebaut.

In den Rahmen der vorliegenden Erfindung fällt auch eine Gehörknöchelchenprothese mit einem ersten Befestigungselement, welches als Kopfplatte der oben beschriebenen erfindungsgemäßen Art ausgebildet ist. Besonders bevorzugt ist die erfindungsgemäß modifizierte Gehörknöchelchenprothese längenvariabel gestaltet. Aufgrund der damit ermöglichten elastischen Verformung kann die Klemmung bei einer Längeneinstellung zu Zwecken einer möglichst präzisen Positionierung sowie der Feinjustage mehrfach wieder gelöst und danach wiederum geklemmt werden.

Je nach dem individuellen Defekt, der bei einem Patienten durch den Einsatz einer mit der erfindungsgemäßen Kopfplatte ausgestatteten Gehörknöchelchenprothese behoben oder zumindest in seinen Auswirkungen gelindert werden soll, wird der Aufbau der Prothese entsprechend gestaltet sein. In sämtlichen Ausführungsformen der Erfindung wird die Kopfplatte zur Anlage am Trommelfell und/oder am Hammergriff ausgebildet sein. Bei vielen Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäß modifizierten Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist. Bei Weiterbildungen dieser Ausführungsformen liegt die Prothese über die Kopfplatte einerseits am Trommelfell und/oder am Hammergriff an und ist über das zweite Befestigungselement andererseits am Amboss oder am Steigbügel befestigt. Alternative Ausgestaltungen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über einen Kolben.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen. Die erfindungsgemäße Kopfplatte der Gehörknöchelchenprothese sollte grundsätzlich eine wachstumsfördernde Beschichtung, ein direkt ins Innenohr führendes, etwa in Form eines Kolbens ausgebildetes zweites Befestigungselement hingegen eine wachstumshemmende Beschichtung aufweisen.

Eine weitere Ausführungsform der Erfindung zeichnet sich schließlich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Prothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

### Detaillierte Beschreibung der Erfindung und Zeichnung

In der schematischen Zeichnung sind Ausführungsbeispiele der Erfindung dargestellt, welche in der nachfolgenden Beschreibung näher erläutert werden.

Es zeigen:
- Fig. 1: eine schematische räumliche Darstellung einer Ausführungsform der erfindungsgemäßen Kopfplatte für eine Gehörknöchelchenprothese in einer Draufsicht schräg von oben;
- Fig. 2a: eine schematische flächige Darstellung einer ähnlichen Ausführungsform wie in Fig. 1 mit Erläuterung der Kräfte und Wege im monolithischen Hebelsystem;
- Fig. 2b: die Ausführungsform von Fig. 2a ohne die Kräfte und Wege; und
- Fig. 2c: Detail A der Ausführungsform nach Fig.2b.

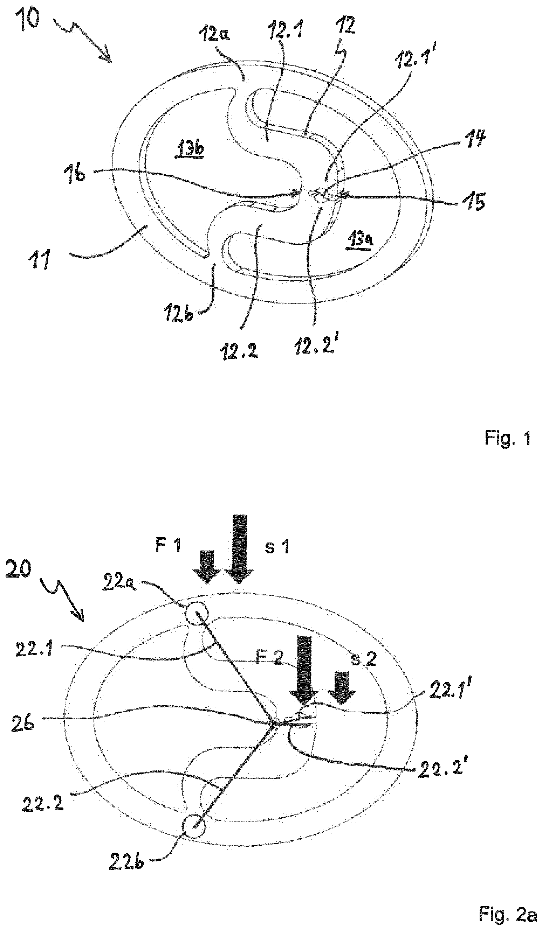

Die in den Figuren 1 bis 2c der Zeichnung schematisch und im Detail dargestellten Ausführungsformen von erfindungsgemäßen **Kopfplatten 10; 20**, die zur mechanischen Anlage von -in der Zeichnung nicht näher dargestellten, vorzugsweise längenvariabel gestalteten-Gehörknöchelchenprothesen am Trommelfell und/oder am Hammergriff ausgebildet sind, können jeweils an einem Ende der betreffenden Gehörknöchelchenprothese als ein erstes Befestigungselement montiert werden. Am anderen Ende der Prothese ist ein zweites Befestigungselement zur mechanischen Verbindung mit einem Glied oder Teilen eines Gliedes der Gehörknöchelchen-Kette oder direkt mit dem Innenohr sowie ein entlang einer Längsachse die beiden Befestigungselemente Schall leitend miteinander verbindendes Verbindungselement mit zumindest einem Schaftförmigen Ende vorgesehen.

Die Kopfplatte 10; 20 weist einen radial äußeren, die Längsachse umgebenden **Ringbereich 11; 21** auf, an dessen Innenrand ein einzelner, längs einer gekrümmten Kurve verlaufender **Steg 12; 22** an zwei voneinander räumlich getrennten **Angriffspunkten 12a,12b; 22a,22b** fest mit dem Ringbereich 11; 21 verbunden ist, wobei seitlich neben dem Steg 12; 22 zwei durch den Steg 12; 22 getrennte **Durchbrüche 13a,13b; 23a,23b** innerhalb des Ringbereichs 11; 21 offen bleiben, wobei in einem mittleren Abschnitt des Stegs 12; 22 eine **Durchgangsbohrung 14; 24** zur Aufnahme des Schaftförmigen Endes des Verbindungselements sowie ein von einem der Durchbrüche 13a; 23a zur Durchgangsbohrung 14; 24 verlaufender **Schlitz 15; 25** zum seitlichen Einführen des Schaftförmigen Endes des Verbindungselements vom Durchbruch 13a; 23a aus in die Durchgangsbohrung 14; 24 vorgesehen ist, und wobei die Durchgangsbohrung 14; 24 nach dem Einführen des Schaftförmigen Verbindungselements verengbar ist, um dieses in der Durchgangsbohrung 14; 24 zu fixieren.

In der Regel werden die Stege 12; 22 geometrisch so gestaltet sein, dass sie bei lokaler Medialbewegung des Trommelfells dieser Medialbewegung lokal folgen, jedoch die Bewegung nicht an entfernte Bereiche der Kopfplatte 10; 20 weitergeben.

Die Erfindung zeichnet sich gegenüber bekannten gattungsgemäßen Kopfplatten von Gehörknöchelchenprothesen dadurch aus, dass der Steg 12; 22 als monolithisches Hebelsystem ausgebildet ist und einen **ersten langen Hebelabschnitt 12.1; 22.1** aufweist, der vom ersten Angriffspunkt 12a; 22a des Steges 12; 22 am Ringbereich 11; 21 zu einer Seite eines im Steg 12; 22 angeordneten gemeinsamen **Biegegelenks 16; 26** verläuft, von dessen anderer Seite ein **zweiter langer Hebelabschnitt 12.2; 22.2** zum zweiten Angriffspunkt 12b; 22b des Steges 12; 22 am Ringbereich 11; 21 verläuft, dass im Steg 12; 22 ein **erster kurzer Hebelabschnitt 12.1'; 22.1',** der eine geringere Länge aufweist als der erste lange Hebelabschnitt 12.1; 22.1, sowie ein **zweiter kurzer Hebelabschnitt 12.2'; 22.2',** der eine geringere Länge aufweist als der zweite lange Hebelabschnitt 12.2; 22.2, vorgesehen sind, wobei der erste kurze Hebelabschnitt 12.1' vom Verbindungspunkt des ersten langen Hebelabschnitts 12.1; 22.1 mit dem gemeinsamen Biegegelenk 16; 26 zu einer ersten Seite des Schlitzes 15; 25 und der zweite kurze Hebelabschnitt 12.2'; 22.2' vom Verbindungspunkt des zweiten langen Hebelabschnitts 12.2; 22.2 mit dem gemeinsamen Biegegelenk 16; 26 zu einer zweiten Seite des Schlitzes 15; 25 verlaufen, und dass der erste Angriffspunkt 12a; 22a des Steges 12; 22 am Ringbereich 11; 21, das gemeinsame Biegegelenk 16; 26 und der zweite Angriffspunkt 12b; 22b des Steges 12; 22 am Ringbereich 11; 21 nicht-kollinear angeordnet sind, so dass durch Betätigung des ersten und zweiten langen Hebelabschnitts 12.1,12.2; 22.1, 22.2 über das gemeinsame Biegegelenk 16; 26 der erste und zweite kurze Hebelabschnitt 12.1', 12.2'; 22.1',22.2' betätigt werden.

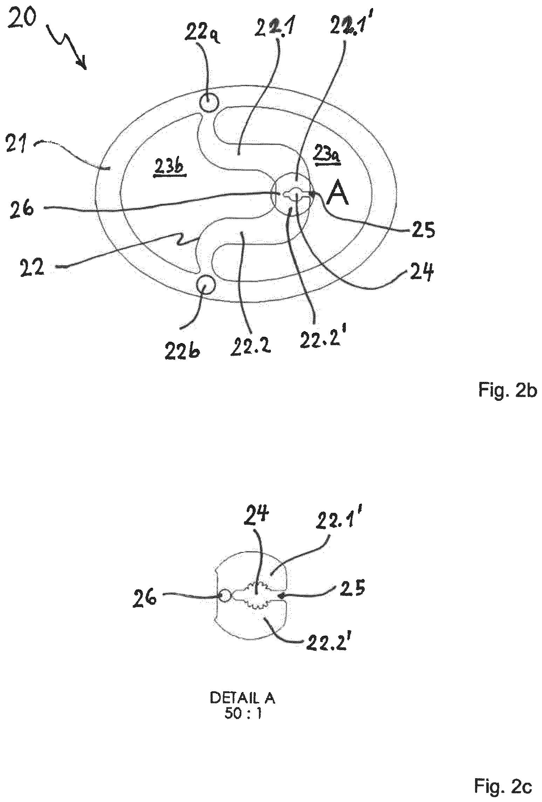

In der schematischen Darstellung von Fig. 2a sind die beiden langen Hebelabschnitte 22.1,22.2 sowie die beiden kurzen Hebelabschnitte 22.1',22.2'jeweils als gerade Linien eingezeichnet. Wie man unmittelbar erkennen kann, wirkt die monolithische Hebelmechanik als Kraftverstärker, indem sie die "Rückstellkraft" der Kopfplatte verstärkt und diese auf den Schaft überträgt, wodurch eine relativ hohe Flächenpressung und somit eine sehr feste Klemmung entsteht. Durch Druck auf die Außenkontur wird auf diese Weise durch die Hebelübersetzung ein großer Weg s1 mit geringer Kraft F1 auf einen sehr kleinen Weg s2 mit großer Kraft F2 übersetzt. Die auf diese Weise auf die Hebelmechanik übertragenen Kräfte sorgen für eine sichere Klemmung der Kopfplatte am Schaft.

Der erste und der zweite lange Hebelabschnitt 12.1,12.2; 22.1,22.2 sind jeweils einstückig am ersten bzw. zweiten Angriffspunkt 12a bzw. 12b; 22a bzw. 22b des Steges 12; 22 mit dem Ringbereich 11; 21 verbunden.

Der erste und der zweite Angriffspunkt 12a, 12b; 22b des Steges 12; 22 am Ringbereich 11; 21 können jeweils als weitere Biegeelemente ausgebildet sein. Insbesondere können der erste und der zweite lange Hebelabschnitt 12.1,12.2; 22.1,22.2 jeweils in einem Bereich vor dem ersten bzw. dem zweiten Angriffspunkt 12a bzw. 12b; 22a bzw. 22b eine geringere Stegbreite aufweisen als in ihren übrigen Bereichen.

Bei den in Zeichnung dargestellten Ausführungsformen der erfindungsgemäßen Kopfplatte 10; 20 ist der Steg 12; 22 in einem Bereich zwischen dem ersten Angriffspunkt 12a; 22a und dem zweiten Angriffspunkt 12b; 22b am Ringbereich 11; 21, der auch das gemeinsame Biegegelenk 16; 26 umfasst, jeweils im Wesentlichen U-förmig gestaltet.

Wie besonders gut in der Detail-Darstellung von Fig. 2c zu erkennen ist, kann der Schlitz 15; 25 im Steg 12; 22 auch jenseits der Durchgangsbohrung 14; 24 bis zum gemeinsamen Biegegelenk 16; 26 fortgesetzt sein.

Bei den hier gezeigten Ausführungsformen ist der radial äußere, die Längsachse umgebende Ringbereich 11; 21 jeweils oval gestaltet. Er kann aber -je nach individuellem Einsatzzweck der erfindungsgemäßen Kopfplatte- auch andere geometrische Formen aufweisen, etwa eine kreisrunde oder sogar eine polygonale Gestaltung.

Wie wiederum in der Detail-Zeichnung von Fig. 2c gut zu erkennen ist, kann die Durchgangsbohrung 14; 24 zur Erhöhung der reibenden Haltekräfte auf den Schaft im eingebauten Zustand auf ihrem Umfang gezackt gestaltet sein.

Der erste bzw. der zweite lange Hebelabschnitt 12.1 bzw. 12.2; 22.1 bzw. 22.2 werden in der Regel zum ersten bzw. zum zweiten kurzen Hebelabschnitt 12.1',12.2'; 22.1',22.2' jeweils ein Hebelverhältnis größer 1,5, vorzugsweise zwischen 2 und 6, aufweisen.

Hinsichtlich der Gestaltung der absoluten Größen von Teilen der erfindungsgemäßen Kopfplatte bestehen weite Freiräume. In der Praxis haben sich folgende Gestaltungen und Größenauswahlen bewährt:
Die Durchgangsbohrung 14; 24 weist einen lichten Durchmesser zwischen 0,1mm und 0,4mm, vorzugsweise von etwa 0,2mm auf.

Der Schlitz 15; 25 im Steg 12; 22 der Kopfplatte 10; 20 weist eine Schlitzbreite zwischen 0,03mm und 0,15mm, vorzugsweise von etwa 0,06mm auf.

Die Kopfplatte 10; 20 Weist eine Plattendicke zwischen 0,1mm und 0,5mm, vorzugsweise von etwa 0,25mm auf.

Vorteilhafterweise sollte die Kopfplatte 10; 20 aus einem hochfesten und elastischen Material mit einer minimalen Festigkeit ≥ 450N/mm² sowie einer minimalen Elastizität ≥ 0,5%, vorzugsweise ≥ 2%, aufgebaut sein.

Vorzugsweise ist die erfindungsgemäße Kopfplatte 10; 20
aus einem Metall oder einer Metalllegierung, insbesondere aus Nitinol
und/oder aus amorphem Metallglas
und/oder aus Metallkeramik
und/oder aus einem hochfesten und elastischen Polymer, insbesondere
aus faserverstärktem Kunststoff, vorzugsweise aus Karbonfaser-Material aufgebaut.

## Patentansprüche

1. Kopfplatte (10; 20) einer Gehörknöchelchenprothese, die mindestens ein Glied oder Teile eines Gliedes der Gehörknöchelchenkette ersetzt oder überbrückt, wobei die Kopfplatte (10; 20) zur mechanischen Anlage am Trommelfell und/oder am Hammergriff ausgebildet ist und an einem Ende der Gehörknöchelchenprothese als ein erstes Befestigungselement montierbar ist, während am anderen Ende der Prothese ein zweites Befestigungselement zur mechanischen Verbindung mit einem Glied oder Teilen eines Gliedes der Gehörknöchelchen-Kette oder direkt mit dem Innenohr sowie ein entlang einer Längsachse die beiden Befestigungselemente Schall leitend miteinander verbindendes Verbindungselement mit zumindest einem Schaftförmigen Ende vorgesehen ist, wobei die Kopfplatte (10; 20) einen radial äußeren, die Längsachse umgebenden Ringbereich (11; 21) aufweist, an dessen Innenrand ein einzelner, längs einer gekrümmten Kurve verlaufender Steg (12; 22) an zwei voneinander räumlich getrennten Angriffspunkten (12a,12b; 22a,22b) fest mit dem Ringbereich (11; 21) verbunden ist, wobei seitlich neben dem Steg (12; 22) zwei durch den Steg (12; 22) getrennte Durchbrüche (13a,13b; 23a,23b) innerhalb des Ringbereichs (11; 21) offen bleiben, wobei in einem mittleren Abschnitt des Stegs (12; 22) eine Durchgangsbohrung (14; 24) zur Aufnahme des Schaftförmigen Endes des Verbindungselements sowie ein von einem der Durchbrüche (13a; 23a) zur Durchgangsbohrung (14; 24) verlaufender Schlitz (15; 25) zum seitlichen Einführen des Schaftförmigen Endes des Verbindungselements vom Durchbruch (13a; 23a) aus in die Durchgangsbohrung (14; 24) vorgesehen ist, wobei die Durchgangsbohrung (14; 24) nach dem Einführen des Schaftförmigen Verbindungselements verengbar ist, um dieses in der Durchgangsbohrung (14; 24) zu fixieren,
**dadurch gekennzeichnet,**
**dass** der Steg (12; 22) als monolithisches Hebelsystem ausgebildet ist und einen ersten langen Hebelabschnitt (12.1; 22.1) aufweist, der vom ersten Angriffspunkt (12a; 22a) des Steges (12; 22) am Ringbereich (11; 21) zu einer Seite eines im Steg (12; 22) angeordneten gemeinsamen Biegegelenks (16; 26) verläuft, von dessen anderer Seite ein zweiter langer Hebelabschnitt (12.2; 22.2) zum zweiten Angriffspunkt (12b; 22b) des Steges (12; 22) am Ringbereich (11; 21) verläuft,
**dass** im Steg (12; 22) ein erster kurzer Hebelabschnitt (12.1'; 22.1'), der eine geringere Länge aufweist als der erste lange Hebelabschnitt (12.1; 22.1), sowie ein zweiter kurzer Hebelabschnitt (12.2'; 22.2'), der eine geringere Länge aufweist als der zweite lange Hebelabschnitt (12.2; 22.2), vorgesehen sind, wobei der erste kurze Hebelabschnitt (12.1') vom Verbindungspunkt des ersten langen Hebelabschnitts (12.1; 22.1) mit dem gemeinsamen Biegegelenk (16; 26) zu einer ersten Seite des Schlitzes (15; 25) und der zweite kurze Hebelabschnitt (12.2'; 22.2') vom Verbindungspunkt des zweiten langen Hebelabschnitts (12.2; 22.2) mit dem gemeinsamen Biegegelenk (16; 26) zu einer zweiten Seite des Schlitzes (15; 25) verlaufen,
und **dass** der erste Angriffspunkt (12a; 22a) des Steges (12; 22) am Ringbereich (11; 21), das gemeinsame Biegegelenk (16; 26) und der zweite Angriffspunkt (12b; 22b) des Steges (12; 22) am Ringbereich (11; 21) nicht-kollinear angeordnet sind, so dass durch Betätigung des ersten und zweiten langen Hebelabschnitts (12.1,12.2; 22.1, 22.2) über das gemeinsame Biegegelenk (16; 26) der erste und zweite kurze Hebelabschnitt (12.1', 12.2'; 22.1',22.2') betätigt werden.

2. Kopfplatte nach Anspruch 1, **dadurch gekennzeichnet, dass** der erste und der zweite lange Hebelabschnitt (12.1,12.2; 22.1,22.2) jeweils einstückig am ersten bzw. zweiten Angriffspunkt (12a bzw. 12b; 22a bzw. 22b) des Steges (12; 22) mit dem Ringbereich (11; 21) verbunden sind.

3. Kopfplatte nach einem der vorhergehenden Ansprüche, **dadurch gekennzeichnet, dass** der erste und der zweite Angriffspunkt (12a, 12b; 22b) des Steges (12; 22) am Ringbereich (11; 21) jeweils als weitere Biegeelemente ausgebildet sind.

4. Kopfplatte nach Anspruch 3, **dadurch gekennzeichnet, dass** der erste und der zweite lange Hebelabschnitt (12.1,12.2; 22.1,22.2) jeweils in einem Bereich vor dem ersten bzw. dem zweiten Angriffspunkt (12a bzw. 12b; 22a bzw. 22b) eine geringere Stegbreite aufweisen als in ihren übrigen Bereichen.

5. Kopfplatte nach einem der vorhergehenden Ansprüche, **dadurch gekennzeichnet, dass** der Steg (12; 22) in einem Bereich zwischen dem ersten Angriffspunkt (12a; 22a) und dem zweiten Angriffspunkt (12b; 22b) am Ringbereich (11; 21), der auch das gemeinsame Biegegelenk (16; 26) umfasst, im Wesentlichen U-förmig gestaltet ist.

6. Kopfplatte nach einem der vorhergehenden Ansprüche, **dadurch gekennzeichnet, dass** der Schlitz (15; 25) im Steg (12; 22) auch jenseits der Durchgangsbohrung (14; 24) bis zum gemeinsamen Biegegelenk (16; 26) fortgesetzt ist.

7. Kopfplatte nach einem der vorhergehenden Ansprüche, **dadurch gekennzeichnet, dass** der radial äußere, die Längsachse umgebende Ringbereich (11; 21) kreisrund oder oval gestaltet ist.

8. Kopfplatte nach einem der vorhergehenden Ansprüche, **dadurch gekennzeichnet, dass** die Durchgangsbohrung (14; 24) auf ihrem Umfang gezackt gestaltet ist.

9. Kopfplatte nach einem der vorhergehenden Ansprüche, **dadurch gekennzeichnet, dass** der erste bzw. der zweite lange Hebelabschnitt (12.1 bzw. 12.2; 22.1 bzw. 22.2) zum ersten bzw. zum zweiten kurzen Hebelabschnitt (12.1',12.2'; 22.1',22.2') jeweils ein Hebelverhältnis ≥ 1,5, vorzugsweise zwischen 2 und 6, aufweist.

10. Kopfplatte nach einem der vorhergehenden Ansprüche, **dadurch gekennzeichnet, dass** die Durchgangsbohrung (14; 24) einen lichten Durchmesser zwischen 0,1mm und 0,4mm, vorzugsweise von etwa 0,2mm, aufweist.

11. Kopfplatte nach einem der vorhergehenden Ansprüche, **dadurch gekennzeichnet, dass** der Schlitz (15; 25) im Steg (12; 22) eine Schlitzbreite zwischen 0,03mm und 0,15mm, vorzugsweise von etwa 0,06mm, aufweist.

12. Kopfplatte nach einem der vorhergehenden Ansprüche, **dadurch gekennzeichnet, dass** die Kopfplatte (10; 20) eine Plattendicke zwischen 0,1mm und 0,5mm, vorzugsweise von etwa 0,25mm, aufweist.

13. Kopfplatte nach einem der vorhergehenden Ansprüche, **dadurch gekennzeichnet, dass** die Kopfplatte (10; 20) aus einem hochfesten und elastischen Material mit einer minimalen Festigkeit ≥ 450N/mm² sowie einer minimalen Elastizität ≥ 0,5%, vorzugsweise ≥ 2%, aufgebaut ist.

14. Kopfplatte nach Anspruch 13, **dadurch gekennzeichnet, dass** die Kopfplatte (10; 20)
aus einem Metall oder einer Metalllegierung, insbesondere aus Nitinol und/oder aus amorphem Metallglas
und/oder aus Metallkeramik
und/oder aus einem hochfesten und elastischen Polymer, insbesondere aus faserverstärktem Kunststoff, vorzugsweise aus Karbonfaser-Material
aufgebaut ist.

15. Gehörknöchelchenprothese mit einem ersten Befestigungselement, welches als Kopfplatte (10; 20) nach einem der vorhergehenden Ansprüche ausgebildet ist, **dadurch gekennzeichnet, dass** die Gehörknöchelchenprothese längenvariabel gestaltet ist.

## Claims

1. Head plate (10; 20) of an auditory ossicle prosthesis, which replaces or bridges over at least one link or parts of a link of the auditory ossicle chain, in which the head plate (10; 20) is made for mechanically lying on the eardrum and/or the handle of the malleus and may be fitted to one end of the auditory ossicle prosthesis as a first fastening element, whilst at the other end of the prosthesis a second fastening element for mechanically connecting to a link or parts of a link of the auditory ossicle chain or directly to the inner ear as well as a connecting element with at least one shaft-shaped end connecting both fastening elements to each other along a longitudinal axis in a sound-conducting way are provided, in which the head plate (10; 20) has a radially outer ring area (11; 21) surrounding the longitidinal axis, at the inner edge of which a single bridge (12; 22) running along an arched curve is connected firmly to the ring area (11; 21) at two working points (12a, 12b; 22a, 22b) spatially separated from each other, in which next to the bridge (12; 22) two openings (13a, 13b; 23a, 23b) within the ring area (11; 21) separated by the bridge (12; 22) remain open, in which in a middle section of the bridge (12; 22) a through hole (14; 24) for receiving the shaft-shaped end of the connecting element as well as a slot (15; 25) running from one of the openings (13a; 23a) to the through hole (14; 24) for laterally inserting the shaft-shaped end of the connecting element from the opening (13a; 23a) into the through hole (14; 24) is provided, in which the through hole (14; 24) may be narrowed after inserting the shaft-shaped connecting element in order to fix it in the through hole (14; 24),
**characterised in that**
the bridge (12; 22) is made as a monolithic lever system and has a first long lever section (12.1; 22.1), which runs from the first working point (12a; 22a) of the bridge (12; 22) in the ring area (11; 21) to one side of a common bending joint (16; 26) arranged in the bridge (12; 22), from the other side of which a second long lever section (12.2; 22.2) runs to the second working point (12b; 22b) of the bridge (12; 22) in the ring area (11; 21),
in the bridge (12; 22) a first short lever section (12.1'; 22.1'), which has a smaller length than the first long lever section (12.1; 22.1), as well as a second short lever section (12.2'; 22.2'), which has a smaller length than the second long lever section (12.2; 22.2), are provided, in which the first short lever section (12.1') runs from the connecting point of the first long lever section (12.1; 22.1) with the common bending joint (16; 26) to a first side of the slot (15; 25) and the second short lever section (12.2'; 22.2') runs from the connecting point of the second long lever section (12.2; 22.2) with the common bending joint (16; 26) to a second side of the slot (15; 25),
and the first working point (12a; 22a) of the bridge (12; 22) in the ring area (11; 21), the common bending joint (16; 26) and the second working point (12b; 22b) of the bridge (12; 22) in the ring area (11; 21) are arranged non-collinear, so that the first and second short lever section (12.1', 12.2'; 22.1',22.2') are operated by operating the first and second long lever section (12.1, 12.2; 22.1, 22.2) through the common bending joint (16; 26).

2. Head plate according to claim 1, **characterised in that** the first and second long lever section (12.1, 12.2; 22.1, 22.2) are connected in one piece to the ring area (11; 21) at the first or second working point (12a or 12b; 22a or 22b) of the bridge (12; 22).

3. Head plate according to one of the previous claims, **characterised in that** the first and second working point (12a, 12b; 22b) of the bridge (12; 22) in the ring area (11; 21) are made as further bending elements.

4. Head plate according to claim 3, **characterised in that** the first and second long lever section (12.1, 12.2; 22.1, 22.2) have a smaller bridge width in an area in front of the first or second working point (12a or 12b; 22a or 22b) than in their other areas.

5. Head plate according to one of the previous claims, **characterised in that** the bridge (12; 22) is made essentially U-shaped in an area between the first working point (12a; 22a) and the second working point (12b; 22b) in the ring area (11; 21), which also comprises the common bending joint (16; 26).

6. Head plate according to one of the previous claims, **characterised in that** the slot (15; 25) in the bridge (12; 22) is also continued beyond the through hole (14; 24) as far as the common bending joint (16; 26).

7. Head plate according to one of the previous claims, **characterised in that** the radially outer ring area (11; 21) surrounding the longitudinal axis is made circular or oval.

8. Head plate according to one of the previous claims, **characterised in that** the through hole (14; 24) is made jagged on its circumference.

9. Head plate according to one of the previous claims, **characterised in that** the first or second long lever section (12.1 or 12.2; 22.1 or 22.2) has a lever ratio of ≥ 1.5, preferably between 2 and 6, to the first or second short lever section (12.1', 12.2'; 22.1', 22.2').

10. Head plate according to one of the previous claims, **characterised in that** the through hole (14; 24) has a clear diameter of between 0.1 mm and 0.4 mm, preferably about 0.2 mm.

11. Head plate according to one of the previous claims, **characterised in that** the slot (15; 25) in the bridge (12; 22) has a slot width of between 0.03 mm and 0.15 mm, preferably about 0.06 mm.

12. Head plate according to one of the previous claims, **characterised in that** the head plate (10; 20) has a plate thickness of between 0.1 mm and 0.5 mm, preferably about 0.25 mm.

13. Head plate according to one of the previous claims, **characterised in that** the head plate (10; 20) is made of a high-strength and elastic material with a minimum strength of ≥ 450 N/mm² as well as a minimum elasticity of ≥ 0.5%, preferably ≥ 2%.

14. Head plate according to claim 13, **characterised in that** the head plate (10; 20) is made of
a metal or a metal alloy, particularly nitinol,
and/or amorphous metal glass
and/or metal ceramic
and/or a high-strength and elastic polymer,
particularly fibre-reinforced plastic, preferably carbon fibre material.

15. Auditory ossicle prosthesis with a first fastening element, which is made as a head plate (10; 20) according to one of the previous claims, **characterised in that** the auditory ossicle prosthesis is made variable in length.

## Revendications

1. Plaque supérieure (10 ; 20) d'une prothèse d'osselet qui remplace ou compense au moins un membre ou des parties d'un membre de la chaîne des osselets, dans laquelle la plaque supérieure (10 ; 20) est conçue pour la pose mécanique au niveau du tympan et/ou du marteau et peut être montée à une extrémité de la prothèse d'osselet en tant que premier élément de fixation, tandis qu'à l'autre extrémité de la prothèse est prévu un second élément de fixation pour la liaison mécanique avec un membre ou des parties d'un membre de la chaîne des osselets ou directement avec l'oreille interne ainsi qu'un élément de liaison reliant l'un à l'autre le long d'un axe longitudinal les deux éléments de fixation de façon à conduire le son avec au moins une extrémité en forme de tige, dans laquelle la plaque supérieure (10 ; 20) présente une région annulaire (11 ; 21) extérieure entourant au niveau radial l'axe longitudinal, au bord intérieur duquel une traverse (12 ; 22) évoluant sur le plan longitudinal d'une courbe incurvée au niveau de deux points d'engagement (12a, 12b ; 22a, 22b) séparés spatialement l'un de l'autre est reliée fixement avec la région annulaire (11 ; 21), dans laquelle latéralement à côté de la traverse (12; 22) deux perforations distinctes (13a, 13b ; 23a, 23b) restent ouvertes au travers de la traverse (12 ; 22) à l'intérieur de la région annulaire (11 ; 21), dans laquelle dans un segment médian de la traverse (12 ; 22), un alésage traversant (14 ; 24) est prévu pour la réception de l'extrémité en forme de tige de l'élément de liaison ainsi qu'une fente (15 ; 25) évoluant depuis l'une des perforations (13a ; 23a) au travers de l'alésage traversant (14 ; 24) pour l'introduction latérale de l'extrémité en forme de tige de l'élément de liaison depuis la perforation (13a ; 23a) dans l'alésage traversant (14 ; 24), dans laquelle l'alésage traversant (14 ; 24) peut être rétrécit après l'introduction de l'élément de liaison en forme de tige pour fixer celui-ci dans l'alésage traversant (14 ; 24),
**caractérisée en ce que**
la traverse (12; 22) est conçue comme un système de levier monobloc et présente un premier segment de levier long (12.1 ; 22.1) qui évolue du premier point d'engagement (12a ; 22a) de la traverse (12 ; 22) au niveau de la région annulaire (11 ; 21) jusqu'à un côté d'une articulation de flexion commune (16; 26) disposée dans la traverse (12 22), de l'autre côté duquel évolue un second segment de levier long (12.2 ; 22.2) jusqu'au second point d'engagement (12b; 22b) de la traverse (12; 22) au niveau de la région annulaire (11 ; 21),
dans la traverse (12 ; 22) sont prévus un premier segment de levier court (12.1' ; 22.1') qui présente une longueur plus réduite que le premier segment de levier long (12.1 ; 22.1), ainsi qu'un second segment de levier court (12.2'; 22,2') qui présente une longueur plus réduite que le second segment de levier long (12.2 ; 22.2), dans laquelle le premier segment de levier court (12.1') évolue du point de liaison du premier segment de levier long (12.1 ; 22.1) avec l'articulation de flexion commune (16 ; 26) jusqu'à un premier côté de la fente (15 ; 25) et le second segment de levier court (12.2' ; 22.2') évolue du point de liaison du second segment de liaison long (12.2 ; 22.2) avec l'articulation de flexion commune (16 ; 26) jusqu'à un second côté de la fente (15 ; 25),
et le premier point d'engagement (12a ; 22a) de la traverse (12 ; 22) au niveau de la région annulaire (11 ; 21), l'articulation de flexion commune (16 ; 26) et le second point d'engagement (12b ; 22b) de la traverse (12 ; 22) au niveau de la région annulaire (11 ; 21) sont disposés de façon non co-linéaire, de sorte que par l'actionnement des premier et second segments de levier longs (12.1, 12.2 ; 22.1, 22.2), par le biais de l'articulation de flexion commune (16 ; 26), les premier et second segments de levier courts (12.1', 12.2'; 22.1', 22.2') sont actionnés.

2. Plaque supérieure selon la revendication 1, **caractérisée en ce que** les premier et second segments de levier longs (12.1, 12.2 ; 22.1, 22.2) sont reliés respectivement d'un seul tenant au niveau du premier ou second point d'engagement (12a ou 12b ; 22a ou 22b) de la traverse (12 ; 22) avec la région annulaire (11 ; 21).

3. Plaque supérieure selon l'une quelconque des revendications précédentes, **caractérisée en ce que** les premier et second points d'engagement (12a, 12b ; 22b) de la traverse (12 ; 22) sont conçus au niveau de la région annulaire (11 ; 21) respectivement en tant qu'autres éléments de flexion.

4. Plaque supérieure selon la revendication 3, **caractérisée en ce que** les premier et second segments de levier long (12.1, 12.2; 22.1, 22.2) présentent respectivement dans une région avant le premier ou le second point d'engagement (12a ou 12b ; 22a ou 22b) une largeur de traverse plus réduite que dans leurs autres régions.

5. Plaque supérieure selon l'une quelconque des revendications précédentes, **caractérisée en ce que** la traverse (12 ; 22) dans une région entre le premier point d'engagement (12a ; 22a) et le second point d'engagement (12b ; 22b) au niveau de la région annulaire (11; 21) qui comprend également l'articulation de flexion commune (16; 26) a une configuration sensiblement en forme de U.

6. Plaque supérieure selon l'une quelconque des revendications précédentes, **caractérisée en ce que** la fente (15 ; 25) dans la traverse (12 ; 22) se poursuit également au-delà de l'alésage traversant (14; 24) jusqu'à l'articulation de flexion commune (16 ; 26).

7. Plaque supérieure selon l'une quelconque des revendications précédentes, **caractérisée en ce que** la région annulaire (11 ; 21) extérieure entourant radialement l'axe longitudinal a une configuration circulaire ou ovale.

8. Plaque supérieure selon l'une quelconque des revendications précédentes, **caractérisée en ce que** l'alésage traversant (14 ; 24) a une configuration dentelée sur son pourtour.

9. Plaque supérieure selon l'une quelconque des revendications précédentes, **caractérisée en ce que** le premier ou le second segment de levier long (12.1 ou 12.2 ; 22.1 ou 22.2) présente par rapport au premier ou au second segment de levier court (12.1', 12.2' ; 22.1', 22.2') respectivement un rapport de levier ≥ 1,5, de préférence entre 2 et 6.

10. Plaque supérieure selon l'une quelconque des revendications précédentes, **caractérisée en ce que** l'alésage traversant (14 ; 24) présente un diamètre intérieur entre 0,1 mm et 0,4 mm, de préférence d'environ 0,2 mm.

11. Plaque supérieure selon l'une quelconque des revendications précédentes, **caractérisée en ce que** la fente (15 ; 25) dans la traverse (12 ; 22) présente une largeur de fente entre 0,03 mm et 0,15 mm, de préférence d'environ 0,06 mm.

12. Plaque supérieure selon l'une quelconque des revendications précédentes, **caractérisée en ce que** la plaque supérieure (10 ; 20) présente une épaisseur de plaque entre 0,1 mm et 0,5 mm, de préférence d'environ 0,25 mm.

13. Plaque supérieure selon l'une quelconque des revendications précédentes, **caractérisée en ce que** la plaque supérieure (10 ; 20) est constituée d'un matériau hautement résistant et élastique comportant une résistance minimale ≥ 450 N/mm² ainsi qu'une élasticité minimale ≥ 0,5 %, de préférence ≥ 2 %.

14. Plaque supérieure selon la revendication 13, **caractérisée en ce que** la plaque supérieure (10; 20) est constituée d'un métal ou d'un alliage métallique, en particulier de Nitinol et/ou de verre métallique amorphe et/ou de matériau céramo-métallique et/ou d'un polymère hautement résistant et élastique, en particulier de matière plastique renforcée par des fibres, de préférence de matériau de fibres de carbone.

15. Prothèse d'osselet comportant un premier élément de fixation qui est conçu en tant que plaque supérieure (10 ; 20) selon l'une quelconque des revendications précédentes, **caractérisée en ce que** la prothèse d'osselet a une configuration de longueur variable.
